(19) Europäisches Patentamt / European Patent Office / Office européen des brevets

(11) **EP 2 238 990 A2**

(12) **EUROPEAN PATENT APPLICATION**

(43) Date of publication:
**13.10.2010 Bulletin 2010/41**

(51) Int Cl.:
*A61L 15/26* (2006.01)    *A61L 15/58* (2006.01)

(21) Application number: **10250728.2**

(22) Date of filing: **07.04.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **07.04.2009 JP 2009093372**

(71) Applicant: **Nitto Denko Corporation**
**Ibaraki-shi, Osaka 567-8680 (JP)**

(72) Inventors:
• **Makabe, Miki**
**Ibaraki-shi**
**Osaka 567-8680 (JP)**

• **Kasahara, Tsuyoshi**
**Ibaraki-shi**
**Osaka 567-8680 (JP)**
• **Hamada, Atsushi**
**Ibaraki-shi**
**Osaka 567-8680 (JP)**
• **Ishikuba, Jun**
**Ibaraki-shi**
**Osaka 567-8680 (JP)**

(74) Representative: **Duckworth, Timothy John**
**J.A. Kemp & Co.**
**14 South Square**
**Gray's Inn**
**London WC1R 5JJ (GB)**

(54) **Patch and patch preparation**

(57) Provided are a patch and a patch preparation having an adhesive layer on at least one surface of a support, and particularly, a patch and a patch preparation superior in skin adhesive force, and causing less adhesive residue on the skin surface. A patch having a support and an adhesive layer formed on at least one surface of the support, wherein the adhesive layer contains an elastomer obtained by crosslinking a polymer having a butadiene skeleton in the presence of an organic peroxide, and the ratio of the weight of a toluene-insoluble component in the adhesive layer to the total weight of the adhesive layer is 5 - 32 wt%.

EP 2 238 990 A2

**Description**

Technical Field

The present invention relates to a patch and a patch preparation having an adhesive layer on at least one surface of a support, and particularly relates to a patch and a patch preparation superior in skin adhesive force, and causing less adhesive residue on the skin surface.

Background Art

Patch is a convenient and effective dosage form for wound protection or administration of a drug to living organisms. Patch is requested to have good adhesive property while stably maintaining the quality for a long time, such as enhanced skin adhesive force, reduced adhesive residue on the skin surface and the like.
As a patch aiming at meeting such requirements, one containing an acrylic adhesive and a crosslinked adhesive layer is disclosed (patent document 1).
However, in a patch based on conventional acrylic adhesives produced for the purpose of maintaining long-term quality and improving adhesive property of the patch, drugs sometimes show low solubility in the adhesive depending on the kind of the drug. Thus, such patch is not sufficiently satisfactory in practical aspects when used as a patch preparation, and has room for improvement.

[prior art document]

[patent document]

patent document 1: JP-A-2003-62058

[SUMMARY OF THE INVENTION]

Problems to be Solved by the Invention

The present invention has been made in view of the above-mentioned situation, and aims to solve the problem of provision of a patch and a patch preparation having good adhesive property such as enhanced skin adhesive force, reduced adhesive residue on the skin surface and the like, which maintain the quality of the patch stably for a long time.
Means of Solving the Problems

The present inventors have conducted intensive studies and found that the presence of an elastomer obtained by crosslinking a polymer having a butadiene skeleton in the presence of an organic peroxide in an adhesive layer and a ratio of the weight of a toluene-insoluble component in the adhesive layer relative to the total weight of the adhesive layer, which is within a predetermined range, are effective for maintaining the quality of a patch stably for a long time. Furthermore, they have found that a patch having such an adhesive layer has superior practicality based on good adhesive property such as enhanced skin adhesive force, decreased adhesive residue on the skin surface and the like, which resulted in the completion of the present invention. Accordingly, the present invention provides the following.

[1] A patch comprising a support and an adhesive layer formed on at least one surface of the support, wherein the aforementioned adhesive layer comprises an elastomer obtained by crosslinking a polymer having a butadiene skeleton in the presence of an organic peroxide, and the ratio of the weight of a toluene-insoluble component in the aforementioned adhesive layer to the total weight of the aforementioned adhesive layer is 5 - 32 wt%.
[2] The patch of the above-mentioned [1], wherein the aforementioned polymer having a butadiene skeleton is polybutadiene.
[3] The patch of the above-mentioned [1] or [2], wherein the aforementioned organic peroxide is dibenzoyl peroxide.
[4] The patch of any of the above-mentioned [1] to [3],
wherein the aforementioned adhesive layer further comprises a tackifier.
[5] The patch of any of the above-mentioned [1] to [4], wherein the aforementioned adhesive layer further comprises an organic liquid component.
[6] The patch of the above-mentioned [5], wherein the aforementioned organic liquid component is isopropyl myristate.
[7] A patch preparation comprising the patch of any of the above-mentioned [1] to [6], wherein the adhesive layer contains a drug.

Effect of the Invention

According to the present invention, a patch with good adhesive property such as superior skin adhesive force, reduced adhesive residue on the skin surface and the like, which maintains the quality of the patch stably for a long time, can be obtained. Moreover, a patch preparation thereof can reduce undesired reactions of a drug and an adhesive composition while maintaining good adhesive property, and can maintain the quality of the drug stably for a long time. Furthermore, by setting the ratio of the weight of the toluene-insoluble component in the adhesive layer to the total weight of the adhesive layer to fall within the range of 5 - 32 wt%, higher cohesion strength than that of acrylic adhesives can be obtained while ensuring an adhesive force necessary for a patch.

[Embodiment of the Invention]

While preferable embodiments of the present invention are shown in the following, the detailed explanation and Examples are solely for illustration purposes, and do not limit the scope of the present invention.

The patch of the present invention comprises a support and an adhesive layer formed on at least one surface of the support, wherein the adhesive layer comprises an elastomer obtained by crosslinking a polymer having a butadiene skeleton in the presence of an organic peroxide, and the ratio of the weight of a toluene-insoluble component in the adhesive layer to the total weight of the adhesive layer is 5 - 32 wt%.

Therefore, the adhesive layer contains an elastomer having a hydrocarbon chain with a comparatively high molecular weight, which affords adhesive force and cohesive force preferable for a patch.

In the present invention, moreover, since a polymer having a butadiene skeleton is used as a basic polymer, introduction of a crosslinkable functional group is not necessary, which enables long-term maintenance of stable quality of a patch during production and storage. Moreover, various basic polymers can be selected without limitation by the functional group introduced into the basic polymer, and the degree of freedom of the selection of the basic polymer can be increased.

In the present invention, the basic polymer optionally further has, in addition to a butadiene skeleton, a hydrocarbon chain from the aspects of stability. The hydrocarbon chain includes a nonaromatic hydrocarbon chain and an aromatic hydrocarbon chain. As the nonaromatic hydrocarbon chain includes alkylene, alkenylene, alkynylene and the like can be mentioned, and as the aromatic hydrocarbon chain, phenylene, naphthylene and the like can be mentioned. When a polymer having a hydrocarbon chain moiety is used, since an elastomer formed by crosslinking has a hydrocarbon chain moiety with a comparatively high molecular weight, and a structure wherein hydrocarbon chains are three-dimensionally entangled intrinsically, the skin adhesive force and cohesive force necessary as a patch can be imparted to the adhesive layer. The hydrocarbon chain may be any of aliphatic, aromatic and alicyclic, and only needs to be present in at least one of the main chain and the side chain.

Examples of specific basic polymer particularly include polybutadiene, styrene-butadiene block copolymer (hereinafter sometimes to be abbreviated as "SB"), styrene-butadienestyrene block copolymer (hereinafter sometimes to be abbreviated as "SBS") and the like. From the aspects of adhesive property, polybutadiene is preferable. While the amount of use is not particularly limited, the ratio thereof to the total weight of the adhesive layer is generally 10 - 75 wt%, preferably 20 - 50 wt%. In the present invention, one or more kinds of such basic polymers can be used in combination depending on the object.

While the weight average molecular weight of the basic polymer is not particularly limited, it is preferably 50,000 - 5,000,000. Here, the "weight average molecular weight" is measured by gel permeation chromatography under the following conditions.
(Analysis conditions)
GPC apparatus: HLC8120 (manufactured by Tosoh Corporation)
column: TSK gel GMH-H(S) (manufactured by Tosoh Corporation)
standard: polystyrene
eluent: tetrahydrofuran
flow rate: 0.5 mL/min
measurement temperature: 40°C
detector: differential refractometer

The present invention is characterized by crosslinking a polymer having a butadiene skeleton portion in the presence of an organic peroxide to give an adhesive layer. The organic peroxide is not particularly limited, and an organic peroxide known per se generally used in the polymer chemical field can be used. For example, diacyl peroxide (e.g., dibenzoyl peroxide, diisobutyryl peroxide, di(3,5,5-trimethylhexanoyl)peroxide, dilauroyl peroxide, disuccinate peroxide, peroxyester (e.g., 1,1,3,3-tetramethylbutyl peroxy-2-ethylhexanoate, 2,5-dimethyl-2,5-di(2-ethylhexanoylperoxy)hexane), t-hexylperoxy-2-ethylhexanoate, t-butyl peroxy-2-ethylhexanoate), ketone peroxide (e.g., methylethyl ketone peroxide), peroxy ketal (e.g., 1,1-di(t-butylperoxy)cyclohexane), hydroperoxide (e.g., p-menthane hydroperoxide), dialkyl peroxide (e.g., dicumyl peroxide), peroxy dicarbonate (e.g., di-n-propyl peroxy dicarbonate) and the like can be mentioned.

Among these, from the aspects of reactivity, diacyl peroxide (particularly dibenzoyl peroxide) and peroxyester (particularly 1,1,3,3-tetramethylbutylperoxy-2-ethylhexanoate) are preferable. Particularly, diacyl peroxide is preferable, which reacts with the basic polymer to give an elastomer having an organic group such as alkyl, phenyl, acyl, benzoyl, acyloxy or benzoyloxy, each of which is optionally substituted. In addition, peroxyester reacts with the basic polymer to give an elastomer having an organic group such as alkyl, alkoxy, phenyl, acyl, benzoyl, acyloxy or benzoyloxy, each of which is optionally substituted.

The amount of such organic peroxide is preferably 0.15 - 0.55 part by weight, more preferably 0.2 - 0.5 part by weight, relative to 100 parts by weight of the basic polymer. When it is less than 0.15 part by weight, the cohesive force of the adhesive layer tends to be insufficient. On the other hand, when it exceeds 0.55 part by weight, the adhesive layer tends to become hard, resulting in lower adhesive force and decreased soft feeling.

In the present invention, the ratio of the weight of a toluene-insoluble component in the adhesive layer to the total weight of the adhesive layer is 5 - 32 wt%. The technical significance of employing the ratio relative to the total weight of the adhesive layer for calculation thereof rests in that fact that, by a gel fraction calculation method based solely on the components involved in crosslinking, when the ratio of the components (polymer etc.) involved in crosslinking of the adhesive as a whole changes, the ratio of the toluene-insoluble component in the adhesive as a whole also changes, and adhesive sheets having the same gel fraction may show different adhesive properties. In other words, the adhesive property is influenced by the ratio of the toluene-insoluble component in the whole adhesive layer, rather than the ratio of the toluene-insoluble component in the polymer.

In the present invention, the weight of a toluene-insoluble component in the adhesive layer should be calculated based on the total weight of the adhesive layer. This is because the property of the adhesive layer depends on the ratio of the toluene-insoluble component in the adhesive layer. In contrast, the weight ratio of the organic solvent-insoluble component calculated based on the total weight of the components involved in the crosslinking of the adhesive layer is considered to reflect the property of the components involved in the crosslinking. Therefore, such numerical value is different from the weight ratio of the toluene-insoluble component in the present invention.

By setting the ratio to the total weight of the adhesive layer to fall within the range of 5 - 32 wt%, a crosslinked component having a high molecular weight and a non-crosslinked component not having a high molecular weight can be balanced. As a result, technical effects of superior skin adhesive force and less adhesive residue on the skin surface can be afforded.

In the present specification, the ratio of the weight of the toluene-insoluble component in the adhesive layer to the total weight of the adhesive layer can be measured by the following method.

A patch sheet is cut in a predetermined size, for example, about 30 mm×30 mm, a release liner is removed and the patch is packaged with a 110 mm×110 mm porous fluorine film with average pore size of $0.2\mu$m and thickness 70 $\mu$m. The opening is tied up with a string made of a nitoflon NTF film. This is placed in a 50 ml sample bottle, and filled with about 40 g of toluene. After immersion for one week at room temperature (toluene is exchanged once during the week), the sheet is dried in a dryer at 120°C for 3 hr.

The weight of the adhesive layer is measured before immersion and after drying, and the ratio of the weight of the toluene-insoluble component to the total weight of the adhesive layer is determined by the following formula (hereinafter to be referred to as insoluble component ratio). insoluble component ratio (%)=(weight (g) of adhesive layer after drying/ weight (g) of adhesive layer before immersion)×100

To set the ratio of the weight of the toluene-insoluble component in the adhesive layer to the total weight of the adhesive layer to 5 - 32 wt%, the amount of an organic peroxide is adjusted as mentioned above and the below-mentioned curing step is preferably applied.

In the present invention, a large amount of other components may be added to an adhesive solution to obtain an adhesive layer containing a large amount of other components. Examples of other components to be contained include a tackifier as an adhesiveness imparting agent. When a tackifier is added, the basic polymer is crosslinked in the presence of an organic peroxide, and takes the tackifier into its structure during formation of a three-dimensional net molecular structure as an elastomer. As a result, a large amount of a tackifier can be contained in the adhesive layer, which in turn is considered to improve the balance of adhesive force and cohesive force. Consequently, the patch of the present invention can improve adhesive force and tackiness during adhesion to the skin and reduce adhesive residue on the skin surface.

As the tackifier, one known in the field of patch can be appropriately selected for use. Examples of the tackifier include petroleum resin (e.g., aromatic petroleum resin, aliphatic petroleum resin), terpene resin, rosin resin, coumarone indene resin, styrene resin (e.g., styrene resin, $\alpha$-methylstyrene), hydrogenated petroleum resin (e.g., alicyclic saturated hydrocarbon resin) and the like. Among these, an alicyclic saturated hydrocarbon resin is preferable since it improves preservation stability of other compounds in the adhesive layer, for example, an organic liquid component, a drug and the like.

Tackifier may be a combination of one or more kinds thereof. When two or more kinds are combined for use, for example, resins different in the kind and softening point may be combined.

In the present invention, the ratio ((a):(b)) of (a) the weight of the basic polymer and (b) the weight of the tackifier is not particularly limited. It is preferably 3.0:1 - 1:2.0, more preferably 2.5:1 - 1:1.75. When the ratio of (a) the weight of the basic polymer is higher than this ratio, the adhesive force of the adhesive layer tends to decrease, the cohesive force tends to be too strong, and a soft feeling tends to decrease. When the ratio of (b) the weight of the tackifier is higher than this ratio, the adhesive layer tends to be too soft and becomes sticky.

In addition, as other component to be contained, an adhesive layer may contain a large amount of an organic liquid component. The effect of containing an organic liquid component is that a soft feeling is afforded during adhesion to the skin, irritation during detaching from the skin is reduced, and adhesive residue on the skin surface is reduced. Moreover, when the below-mentioned drug is contained in an adhesive layer, its transdermal absorption can be promoted.

As such organic liquid component, a hydrophobic liquid component such as fatty acid alkylester is preferable in view of compatibility with an adhesive layer. Examples of the fatty acid alkylester include fatty acid alkylester composed of higher fatty acid having a carbon number of 12 - 16, preferably 12 - 14, and monovalent lower alcohol having a carbon number of 1 - 4. As the above-mentioned higher fatty acid, lauric acid (C12), myristic acid (C14) and palmitic acid (C16) can be mentioned, with preference given to myristic acid. As the above-mentioned monovalent alcohol, methyl alcohol, ethyl alcohol, propyl alcohol, butyl alcohol and the like can be mentioned, with preference given to isopropyl alcohol. Thus, preferable fatty acid alkylester is isopropyl myristate. One or more kinds of organic liquid components may be used in combination.

The content of the organic liquid component is preferably 5 - 300 parts by weight, more preferably 50 - 170 parts by weight, relative to 100 parts by weight of the weight of the basic polymer. When the amount of the organic liquid component is not less than 5 parts by weight, a high soft feeling can be imparted to the adhesive layer and, when the adhesive layer contains a drug, a high transdermal absorption promoting effect can be obtained. When it is not less than 50 parts by weight, a superior soft feeling can be imparted to the adhesive layer, while suppressing a decrease in the adhesive force and cohesive force of the whole adhesive layer and, when the adhesive layer contains a drug, high transdermal absorbability is advantageously obtained.

In the present invention, moreover, a drug may be contained in an adhesive layer and a patch preparation can also be produced. As mentioned above, the basic polymer does not require a functional group for crosslinking. Therefore, undesirable reaction in the adhesive layer can be suppressed, thus contributing to the stability of the drug. In addition, an undesired reaction between a drug and the functional group of the basic polymer does not need to be considered, thus increasing the degree of freedom of the design of the patch.

The drug is not particularly limited, and a drug that can be administered to mammals such as human and the like through the skin thereof, i.e., a transdermally absorbable drug, is preferable. Two or more kinds of drugs can be used in combination as necessary. Specific examples of such a drug include anesthetics, hypnotic sedatives, antiepileptic drugs, antipyretic analgesic antiphlogistic drugs, anti-vertiginous drugs, psychoneurotic drugs, topical anesthetics, skeletal muscle relaxants, autonomic drugs, antiepileptic drugs, anti-Parkinsonian drugs, anti-histamine drugs, cardiac stimulants, drugs for arrhythmia, diuretic, hypotensive drug, vasoconstrictor, coronary vasodilator, peripheral vasodilators, arteriosclerosis drugs, drugs for circulatory organ, anapnoics, antitussive expectorant, hormone drugs, external drugs for mattery diseases, analgesic-antipruritic-styptic-antiinflammatory agent drugs, drugs for parasitic dermatic diseases, drugs for arrest of bleeding, gout treatment drugs, drugs for diabetes, anti-malignant tumor drugs, antibiotic, chemical therapy drugs, narcotic, quit smoking aids, and the like.

While the content of the drug is not particularly limited as long as the effect of the transdermally absorbable drug is provided and the adhesive property of the adhesive is not impaired, it is preferably, for example, 0.5 - 50 parts by weight relative to the weight of 60 parts by weight of the basic polymer.

The adhesive layer may additionally contain additives known per se, for example, anti-aging agent, antioxidant, UV absorber, filler and the like as necessary.

While the support to be used in the present invention is not particularly limited, a support substantially impermeable to the adhesive layer components such as adhesive, additive, drug and the like, namely, one that does not permit them to penetrate the support and be lost from the back face of thereof to decrease the content is preferable.

As such support, for example, a single film or a laminate film of polyester, nylon, saran (registered trade mark), polyethylene, polypropylene, polyvinyl chloride, ethylene-ethyl acrylate copolymer, ethylene-vinyl acetate copolymer, polytetrafluoroethylene, surlyn (registered trade mark), film made from ionomer resin etc., metal foil and the like, and the like can be used. The thickness of the support is not particularly limited, and is generally 10 - 500 $\mu$m, preferably 10 - 200 $\mu$m.

To improve the adhesive force (anchor force) between the support and the adhesive layer, the support is preferably a laminate film of a non-porous plastic film and a porous film, both composed of the above-mentioned materials, and the adhesive layer is preferably formed on the porous film side.

Such porous film is not particularly limited as long as it improves an anchoring force with the adhesive layer. Examples thereof include paper, woven fabric, non-woven fabric, knitted fabric, mechanically perforated sheet and the like. Among these, from the aspects of handling property and the like, paper, woven fabric and non-woven fabric are particularly preferable. The fabric weight is not subject to any particular limitation but is generally 5 - 30 g/m$^2$, preferably 6 - 15 g/m$^2$.

A most desirable support in the present invention is a laminate film of a polyester film with 1.5 - 6 $\mu$m thickness (preferably, poly(ethylene terephthalate) film) and a non-woven fabric made of polyester (preferably, poly(ethylene terephthalate)) having a basis weight of 6 - 12 g/m$^2$.

Since the patch and patch preparation of the present invention protect the adhesive face of an adhesive layer before use, a release liner is preferably laminated on the adhesive face. The release liner is not particularly limited as long as it is release-treated and ensures sufficient easy-to-release property. For example, films of polyester, polyvinyl chloride, polyvinylidene chloride, poly(ethylene terephthalate) and the like, papers such as quality paper, glassine paper and the like, or a laminate film of quality paper, glassine paper etc. and polyolefin, and the like after a detach treatment by applying silicone resin, fluorine resin and the like to the surface to be in contact with the adhesive layer can be used. Preferred is polyester. The thickness of the release sheet is generally 10 - 200 $\mu$m, preferably 25 - 100 $\mu$m.

The patch and patch preparation of the present invention can be used by detaching a release liner immediately before use and adhering the exposed adhesive face to the skin surface and the like.

The patch and patch preparation of the present invention are produced, for example, in the following manner. While the production method is not particularly limited, it includes, for example, (i) a step of dissolving or dispersing a polymer having a butadiene skeleton as the basic polymer and, where necessary, an adhesiveness imparting agent, an organic liquid component, a drug and the like in a solvent, (ii) a step of applying the obtained viscous solution or dispersion on at least one surface of a support, and drying same to form an adhesive layer on the surface of the support, and (iii) a step of forming a release liner on the adhesive layer (what is called direct transfer method). Alternatively, a patch and a patch preparation can also be produced by a method including (i) a step of applying the above-mentioned solution or dispersion on at least one surface of a release liner for protection, (ii) a step of drying same to form an adhesive layer on the surface of the release liner, and (iii) a step of adhering a support to the adhesive layer (what is called transcription method).

To set the ratio of the weight of the toluene-insoluble component in the adhesive layer to the total weight of the adhesive layer within a predetermined range, the above-mentioned method preferably further includes, for example, a curing step. The curing step can be performed by heating and storing at, for example, 50 - 300°C for 10 - 100 hr. More preferably, the above-mentioned rate can be effectively controlled by performing the above-mentioned curing step in an atmosphere of oxygen concentration 0.01 - 1 vol%.

The thickness of the adhesive layer of the present invention after drying is preferably 10 $\mu$m - 1000 $\mu$m, particularly preferably 20 $\mu$m - 500 $\mu$m, from the aspects of skin adhesiveness.

The form of the patch and patch preparation of the present invention is not particularly limited and may be, for example, a tape, a sheet, a reservoir type and the like.

The present invention is explained in more detail in the following by referring to Examples, which are not to be construed as limitative.

### Examples

Production Example (patch)

Under an air atmosphere, polybutadiene (weight average molecular weight 458,000, 35 parts by weight) as a basic polymer, alicyclic saturated hydrocarbon resin (softening point 100.5°C, ring-and-ball method, 35 parts by weight) as a tackifier, and isopropyl myristate (30 parts by weight) as an organic liquid component were added to toluene, and they were mixed and stirred. To the obtained mixture was added dibenzoyl peroxide as an organic peroxide at a blending ratio shown in Table 1, and they were mixed and stirred to give an adhesive solution. The amount in parts by weight of dibenzoyl peroxide shown in Table 1 is the amount of dibenzoyl peroxide relative to 100 parts by weight of polybutadiene. The adhesive solution was adjusted to have a solid content of the adhesive solution of 25 - 40 wt%, the adjusted adhesive solution was applied onto a polyester release liner (75 $\mu$m thick) such that the thickness after drying was 100 $\mu$m and dried to form an adhesive layer.

As a support, a lamination film of a polyester film (2 $\mu$m thick) and a polyester non-woven fabric (fabric weight 8 g/m$^2$) was used, and the above-mentioned adhesive layer was laminated on the surface of the non-woven fabric of the support. This was packaged with a packaging material of a laminate film of poly(ethylene terephthalate), aluminum or acrylonitrile resin, and heated and cured in the package in the presence of nitrogen (oxygen concentration 0.1 vol%) at 80°C for 2 days to give the patches of Examples 1 - 6 and Comparative Examples 2 - 4. In Comparative Example 1, a patch was prepared using the amounts shown in Table 1.

Production Example (patch preparation)

In the same manner as in Example 1 - 6 except that 35 parts by weight of polybutadiene was exchanged with 34 parts by weight of polybutadiene and 1 part by weight of indomethacin, the patch preparations of Examples 7 - 12 were produced.

[Experimental Examples]

The patches of Examples 1 - 12 and Comparative Examples 1 - 4 were evaluated based on the measurement of the following evaluation items. The evaluation results of Examples 1 - 6 and Comparative Examples 1 - 4 are shown in Table 1.

(1) Adhesive force

In a room at 23°C, 60%RH, the patch was cut into a test piece (width 12 mm, length 5 cm), a release liner was removed from the test piece, and the test piece was press-bonded to a phenol resin test plate by one reciprocation of a 2 kg roller. The test piece was left standing under such environment for 30 min and the adhesive force was measured by stretching the test piece by a tensile tester at a detach angle of 180° and a detach rate of 300 mm/min. As for the failure mode, the cohesive failure was G and interface failure was K. Here, the average value of the adhesive force in Table 1 is an average of the measurement results of three test pieces, where a test piece having the value of not less than 0.5 (N/12 mm) and free of cohesive failure, i.e., failure mode is interface failure "K", is preferable as an evaluation of adhesive force. In addition, SD means standard deviation.

(2) Insoluble component ratio

A patch sheet was cut in 30 mm×30 mm, a release liner was removed and the adhesive sheet was packaged with a 110 mm×110 mm porous fluorine film with average pore size of 0.2 μm and thickness 70 μm. The opening was tied up with a string made of a nitoflon NTF film. This was placed in a 50 ml sample bottle, and filled with about 40 g of toluene. After immersion for one week at room temperature (toluene was exchanged once during the week), the sheet was dried in a dryer at 120°C for 3 hr.
The weight of the adhesive layer was measured before and after drying, and the insoluble component ratio was determined by the following formula.

$$\text{insoluble component ratio (\%)} = (\text{weight (g) of adhesive layer after drying/weight (g) of adhesive layer before immersion}) \times 100$$

The insoluble component ratio of Table 1 is an average of the measurement results of three test pieces, and is preferably 5 - 32%. SD means standard deviation.
From the property evaluation results in Table 1, the patches of Examples 1 - 6 showed an adhesive force of not less than 0.5 (N/12 mm) and had adhesive force and cohesive force necessary for a patch. The patches of Examples 1 - 6 developed interface failure between the testing plane and the adhesive layer during the adhesive force measurement, and an adhesive residue was not found on the testing plane. In contrast, the patch of Comparative Example 1 does not contain an organic peroxide, and therefore, the basic polymer was not crosslinked, the cohesion strength was insufficient, cohesive failure was developed during the adhesive force measurement, and an adhesive residue was observed. In Comparative Examples 2 - 4, since the amount of dibenzoyl peroxide (organic peroxide) added was too high, the crosslinking reaction proceeded too far, and therefore, the insoluble component ratio was as high as 33 - 35%, which resulted in a low adhesive force. The patch preparations of Examples 7 - 12 showed good property like the patches of Examples 1 - 6.
Table 1

| | | composition (parts by weight) | | | | property evaluation results | | | | |
|---|---|---|---|---|---|---|---|---|---|---|
| | | | | | | adhesive force (N/12 nm) | | | insoluble component ratio (%) | |
| | | polybutadiene | dibenzoyl peroxide | isopropyl myristate | tackifier | average | SD | failure mode | average | SD |
| Ex. 1 | | 35 | 0.20 | 30 | 35 | 1.30 | 0.04 | K | 9 | 0 |
| Ex. 2 | | 35 | 0.25 | 30 | 35 | 0.92 | 0.12 | K | 18 | 1 |
| Ex. 3 | | 35 | 0.30 | 30 | 35 | 0.72 | 0.14 | K | 20 | 0 |
| Ex. 4 | | 35 | 0.35 | 30 | 35 | 0.64 | 0.04 | K | 25 | 0 |
| Ex. 5 | | 35 | 0.40 | 30 | 35 | 0.55 | 0.02 | K | 29 | 0 |
| Ex. 6 | | 35 | 0.50 | 30 | 35 | 0.59 | 0.08 | K | 31 | 0 |
| Com. Ex. 1 | | 60 | 0 | 20 | 20 | 1.00 | 0.02 | G | - | - |
| Com. Ex. 2 | | 35 | 0.60 | 30 | 35 | 0.44 | 0.06 | K | 33 | 0 |
| Com. Ex. 3 | | 35 | 0.70 | 30 | 35 | 0.36 | 0.05 | K | 35 | 0 |
| Com. Ex. 4 | | 35 | 0.80 | 30 | 35 | 0.41 | 0.03 | K | 35 | 0 |

Industrial Applicability

Since the patch of the present invention shows good adhesive property while maintaining the quality stably for a long time, it can also be used for the production of a patch preparation.

**Claims**

1.  A patch comprising a support and an adhesive layer formed on at least one surface of the support, wherein said adhesive layer comprises an elastomer obtained by crosslinking a polymer having a butadiene skeleton in the presence of an organic peroxide, and
the ratio of the weight of a toluene-insoluble component in said adhesive layer to the total weight of said adhesive layer is 5 - 32 wt%.

2.  The patch according to claim 1, wherein said polymer having a butadiene skeleton is polybutadiene.

3.  The patch according to claim 1 or 2, wherein said organic peroxide is dibenzoyl peroxide.

4.  The patch according to any one of claims 1 to 3, wherein said adhesive layer further comprises a tackifier.

5.  The patch according to any one of claims 1 to 4, wherein said adhesive layer further comprises an organic liquid component.

6.  The patch according to claim 5, wherein said organic liquid component is isopropyl myristate.

7.  A patch preparation comprising the patch according to any one of claims 1 to 6, wherein the adhesive layer contains a drug.

**REFERENCES CITED IN THE DESCRIPTION**

**Patent documents cited in the description**

• JP 2003062058 A